# EUROPEAN PATENT SPECIFICATION

(11) **EP 1 180 944 B1**
(45) Date of publication and mention of the grant of the patent: **11.02.2004**
(21) Application number: 00935367.3
(22) Date of filing: 01.06.2000
(51) Int. Cl.: A23L 1/305, A23L 1/30

(54) **COMPOSITIONS CONTAINING CREATINE IN SUSPENSION**
ZUSAMMENSETZUNGEN DIE KREATIN IN FORM EINER SUSPENSION ENTHALTEN
COMPOSITIONS CONTENANT DE LA CREATINE EN SUSPENSION

(30) Priority: 02.06.1999 US 324119; 18.10.1999 US 419922
(43) Date of publication of application: 27.02.2002
(73) Proprietor: The Howard Foundation, Cambridge, Cambridgeshire CB2 5JY (GB)
(72) Inventor: Howard, Alan Norman, Great Shelford, Cambridge CB2 5JY (GB); Harris, Roger Charles, Newmarket, Suffolk CB8 8HD (GB)
(74) Representative: Lipscombe, Martin John
(86) International application number: PCT/GB2000/002091
(87) International publication number: WO 2000/074500

(56) References cited:
- EP-A- 0 370 994
- EP-A- 0 449 787
- EP-A- 0 669 083
- WO-A-00/10408
- WO-A-94/02127
- WO-A-94/15488
- WO-A-97/45026
- WO-A-98/53704
- US-A- 5 726 146

## Description

### Field of the Invention

This invention relates to compositions for human consumption comprising creatine in suspension and to a method of providing stable creatine containing compositions.

### Background to the Invention

In the last few years there has been considerable interest among athletes in creatine, which occurs abundantly in skeletal muscle. Creatine plays a pivotal role in the regulation and homeostasis of skeletal muscle energy metabolism and it is now generally accepted that the maintenance of phospho-creatine availability is important to the continuation of muscle force production. Although creatine synthesis occurs in the liver, kidney and pancreas it has been known for sometime that the oral ingestion of creatine will add to the whole body creatine pool, and it has been shown that the ingestion of 20 to 30g creatine per day for several days can lead to a greater than 20 % increase in human skeletal muscle total creatine content. Thus, WO94/02127 discloses the administration of creatine in amounts of at least 15g (or 0.2-0.4 g/kg body weight) per day, for at least 2 days, for increasing muscular strength.

In fact, it was subsequently found that after several days of supplementation (20g per day) with creatine in order to attain saturation, thereafter it takes no more than 2 to 3g per day to maintain the saturation of body stores. Creatine supplementation in an appropriate dose can provide improvements to athletes involved in explosive events, which include all events lasting from a few seconds to a few minutes (such as sprinting, swimming, weight-lifting etc). Endurance performance in events lasting longer than about 30 minutes appears to be unaffected by creatine supplementation. Creatine is a normal food component and is not a drug and its use is not contrary to official regulations. The biggest benefits of supplementation can be experienced by the elderly, vegetarians or those who eat no meat or fish since these people tend to have low muscle creatine content.

Aloe Vera (Aloe barbadensis) is a member of the lily family and is a cactus-like succulent plant that grows in warm frost-free climates. Central American Mexican Indians used Aloe Vera for centuries as a remedy for burns, to prevent blisters, peptic and duodenal ulcers and all types of stomach and intestinal disorders, kidney infections, topical and gastric ulcers as well as to promote longevity. Today Aloe Vera is becoming very popular and its benefits are scientifically recognized. For clarity, the term "Aloe Vera" as used herein refers to the plant, and the term "Aloe Vera extract" refers to compositions prepared by processing the Aloe Vera (e.g. mashing, pulping, cutting, juicing and the like). Thus, Aloe Vera gel and Aloe Vera juice are examples of Aloe Vera extract.

The main use of Aloe Vera in the past has been to prevent inflammation, particularly to the skin, especially after burns, but there are many other uses. Experiments and research studies have shown that after using Aloe Vera juice, the output of the digestive enzymes and the bacterial population of the intestines are improved. Thus there has been an increasing interest in Aloe Vera extract as a medicament to be taken orally as people become more acquainted with its medicinal properties

Among the several methods of presentation, there is a growing use of Aloe Vera extract in soft drinks which are fruit flavoured, and these are quite palatable. The inclusion of creatine in a soft drink would be highly desirable because the Aloe Vera extract drink would be much more beneficial to health than an unsupplemented ordinary fruit drink.

Aloe Vera juice is acidic (commonly about pH3). It is well known that creatine is unstable in aqueous solutions at acid or neutral pH, and is converted into the related compound creatinine. This is highly significant as creatinine has no muscle performance-enhancing effect and is excreted from the human body as a waste product in urine. In view of the foregoing, EP 0 669 083 teaches that aqueous drinks for human consumption comprising creatine must be weakly alkaline, in order to prevent the conversion of creatine into creatinine, and this has become the generally accepted opinion.

Furthermore, creatine has been used in the past only for the preparation of products with a meaty or savoury flavour. For instance, Tonsbeek (US 3,615,600) discloses and is concerned with artificial flavouring mixtures which can impart a meaty .flavour to foods. Similary de Rooji (US 4, 464, 409) is concerned with meat flavouring. Yamazaki (JP-A-59035663) prepares a meat flavour by heating a mixture comprising creatine at pH 5.0-7.0 at temperature of 80-130°C for 30 to 120 minutes. Under these conditions most of the creatine would be converted to creatinine.

Further compositions containing creatine are known from e.g., WO 9745026, WO 9402127, WO 0010408, EP 0 669 083, and EP 0 370 994.

It would be a great advantage to present a composition for human consumption, in which the creatine therein was substantially stable, even at acidic pH and at ambient temperatures.

### Summary of the Invention

In a first aspect the invention provides a composition for human consumption comprising creatine suspended in an edible supporting matrix. The term "suspended" is intended to mean that compositions in accordance with the invention comprise creatine in solid form (e.g. as crystals, powder or the like), distributed within an edible viscous liquid or semi-liquid, or a solid, supporting matrix, typically such that settling (under the influence of gravity) of the solid creatine is inhibited or (preferably) prevented.

The creatine content of the composition may be present as any active form of creatine (e.g. creatine phosphate), but creatine monohydrate is found particularly convenient as a source of creatine. The creatine content of the composition is preferably subjected to a micronization process (e.g. crushing, pulverizing, powdering and the like) prior to incorporation into the matrix, so that the resulting composition is not unacceptably "gritty" in texture.

The composition may be provided in solid, liquid or semi-liquid form (e.g. as a drink, soup or yogurt).

Preferably the creatine will be distributed substantially evenly throughout the supporting matrix (by homogenizing in some manner e.g. by stirring, blending and the like), which may be accomplished manually (e.g. by the consumer) and/or mechanically at the time the composition is prepared.

Conveniently the supporting matrix is a recognised foodstuff, such that a composition in accordance with the invention may take the form of an otherwise conventional foodstuff, supplemented with creatine, such that solid creatine becomes suspended in the foodstuff. Examples of foodstuffs which may represent suitable supporting matrices for the composition of the invention include spreadable solids such as dairy or cheese spreads, margarines, caviar (mainly lump fish caviar) spread, and other fish pastes and spreads (eg. buckling paste i.e. a paste made from smoked baltic herring) meat spreads, and the like. Other convenient supporting matrices are those comprising sugars or other carbohydrates, such as liquid ("runny") or solid ("set") honey, molasses, syrup (e.g. corn syrup, glucose syrup), treacle or "Maxim Energy Gel"™.

If desired, the viscosity of the edible matrix and/or the composition as a whole, may be increased by the addition of viscosifiers, gelling agents and the like. Such components are well-known in the food industry and include, for example, plant-derived or algal-derived polysaccharides, gums and the like, such as galactomannans, dextrans, guar gum, locust bean gum, xanthan gum, carrageen, carrageenan, traganth, acacia, tara, gellan and so on. Other materials suitable for use in the composition include gelatinous substances (e.g. gelatine) or thickening agents such as alginates, agar or pectins.

Indeed, such viscosifiers, gels, gums, thickening agents listed in the preceding paragraph and the like may wholly or partly form the supporting matrix, if desired. One edible matrix comprises a gel prepared from concentrated Aloe Vera extract: a smooth creamy paste (suitable for packaging in a squeezable tube) may be prepared by mixing 5gms of creatine with 20mls of a concentrated Aloe Vera gel (such as that obtainable from Aloe Commodities Int. Inc., Farmers Branch, TX75234). Generally preferred, however, are edible matrices comprising a plant-derived or algal-derived polysaccharide or gum.

The present inventors have previously found that the conversion of creatine to creatinine in acidic aqueous solutions can be markedly inhibited by storage of creatine-containing solutions below ambient temperature. The inventors have now found that, by providing creatine in the form of a suspension, rather than in solution, conversion to creatinine (even in an acidic composition) can be greatly inhibited or even substantially prevented even at ambient (i.e. 20-25°C) temperature. Thus, in some embodiments the composition as a whole (and/or the supporting matrix in isolation) may conveniently be selected to be acidic (i.e. have a pH below 7.0), without significantly adversely affecting the stability of the creatine content of the composition. In particular the composition desirably has a pH between 2.5 and 6.5, preferably between 3.0 and 6.0. Typically the composition has a pH in the range 3.5 to 5.5 which, to the human palate, has a refreshingly sharp taste without being too acidic.

Compositions in accordance with the invention are substantially stable so that creatine may be presented in acidic formulations, contrary to the teaching of the art, in physiologically useful amounts, even following storage for prolonged periods at ambient temperature. A physiologically effective amount of creatine is an amount sufficient to cause a measurable increase in the creatine content of the tissues of a subject following repeated consumption of the composition (e.g. over a 7 day period), relative to an initial baseline level.

The term "substantially stable" is herein defined as referring to a composition in which at least 75 % of the original creatine in the composition is unchanged into creatinine for a period of at least 7 days' storage at ambient (20-25°C) temperature. Desirably the composition will be sufficiently stable that 75% of the creatine remains following a period of at least 31 days', more preferably 45 days', and most preferably at least 73 days' storage at ambient temperature.

The composition may comprise one or more further components to improve its palatability, stability, flavour or nutritive quality. These further components may be selected from the group consisting of: electrolytes, vitamins, lipids, carbohydrates, amino acids, trace elements, colourings, flavours, artificial sweeteners, natural health improving substances, anti-oxidants, stabilizers, preservatives, and buffers. The composition may be unflavoured or where the edible matrix is a recognised foodstuff (e.g. honey or syrup), the composition may have the normal flavour of the matrix. Alternatively, exogenous flavouring may be added (e.g. fruit, cheese or fish flavour).

Vitamins may be included with advantage in the composition of the invention. The following vitamins may be added in amounts which range from 20 to 100% of their recommended daily allowance (RDA). The following are typical of those which are useful: vitamin E, vitamin C, thiamin, riboflavin, niacin, vitamin B6, folacin, vitamin B12, biotin, and pantothenic acid.

In some cases a lipid component may be desirable. The carbohydrate content (if any) or the composition may be present as starch (particularly soluble starch) and/or sugars. The sugars which may be present in the composition include glucose, fructose, sucrose, and maltose.

Artificial sweeteners which can be used include Aspartame, Acesulfam K, Saccharin and Cyclamate. Almost any desired flavoring can be added, most preferably fruity flavors such as berry, lemon, orange, papaya and grapefruit. Citric acid may also be used as an acidulant and citrate (e.g. sodium citrate) as a buffering agent. Also other natural health improving substances may be added in physiologically active amounts. The following are typical of those which are useful: Pan D'Arco tea, Ginseng, Suma tea, Ginkgo, bee pollen, myrrh.

Preservatives can be provided typically by potassium benzoate and/or potassium sorbate.

Colouring can be provided, typically by using a cold water soluble colourant such as beta-carotene. Other suitable colourings will be apparent to those skilled in the art.

A clouding agent may be included in the composition, if desired, to improve the appearance of the composition.

The mineral and trace elements can also be added in any type or form which is suitable for human consumption. It is convenient to provide the calcium and potassium in the form of their gluconates, phosphates or hydrogen phosphates, and magnesium as the oxide or carbonate, chromium as chromium picolinate, selenium as sodium selenite or selenate, and zinc as zinc gluconate. Typically the amounts are:- sodium at 400mg/litre, calcium at 100mg/litre, chloride at 600mg/litre, potassium at 200mg/litre, magnesium at 75mg/litre and phosphorus at 50mg/litre, chromium at 125 g/litre, selenium at 125 g/litre and zinc at 15mg/litre.

The amount of creatine per litre of per Kg of prepared composition may range from 5 to 300g, with a preferred content of about 50g per litre. The normal serving size is in the range 50-330ml, providing about 1-10g (preferably about 3-5g) of creatine. During the first 4 days of creatine supplementation the recommended consumption is 500ml per day divided in 4 or 5 parts per day to achieve creatine saturation. This is followed by 1 serving of 50-250ml per day containing about 3g of creatine to provide a maintenance level of creatine.

In a second aspect the invention provides a method of preparing a creatine containing composition for human consumption in which the creatine is substantially stable, the method comprising the steps of providing creatine in solid form; and mixing the solid creatine with an edible supporting matrix so as to distribute the solid creatine within the supporting matrix. The method typically will comprise the further step of packaging the composition. The composition may be packaged in any of a number of conventional packages (e.g. jars, tins, plastic containers, pots, squeezable tubes and the like).

Advantageously, performance of the method of the second aspect will result in a composition in accordance with the first aspect defined above.

In a third aspect the invention provides a method of storing creatine in stable form, the method comprising the steps of providing creatine in solid form; mixing the solid creatine with an edible supporting matrix so as to suspend the creatine therein; and storing the suspended creatine at ambient temperature or below.

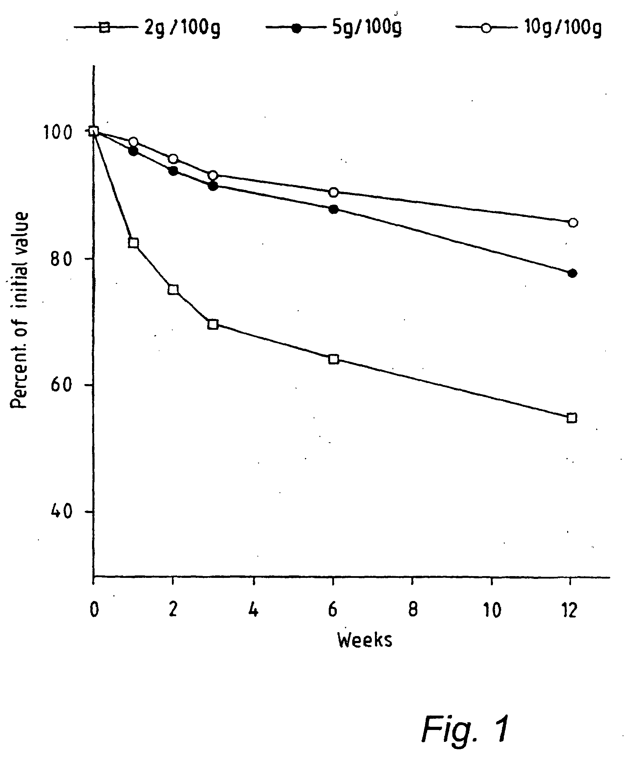

The invention will now be further described by way of illustrative example and with reference to the accompanying drawing (Figure 1) which is a graph of % creatine remaining against time (in weeks).

### Example 1

Four supporting matrices were tested for their suitability for use in the present invention: Sainsbury's liquid honey; Sainsbury's solid honey; Maxim Energy Gel™; and Tate and Lyle Golden Syrup. The Sainsbury's honey and Tate and Lyle syrup are representative of other products of this type. Maxim Energy Gel™ is available from Prinsen BV (Helmond, The Netherlands) and comprises glucose syrup, water, citric acid, flavourings and carotene. Typical composition (per. 100gms) is: carbohydrate 77.6gm; vitamin B1 0.5mg; trace amounts of protein; and water to 100gms.

**Experimental Protocol**
1. The pH of each matrix was determined by dissolving 11g in 50ml distilled water and determining the pH of the resulting solution with a pH meter.
2. 4g of creatine monohydrate was well mixed and suspended in 40g of each matrix to give a concentration of 90.9g/kg of mixture.
3. The mixtures were stored in a dark cabinet at ambient temperature (22.5-23.5°C) in a laboratory.
4. 2-3g of mixture were sampled after 0, 14, 31, 45 and 73 days and stored frozen at -30°C until analysed.
5. Each sample was dissolved in 500ml of distilled water and the creatine concentration was determined by the method of Harris *et al*, (1974 Scand. J. Clin. Lab. Invest. **33**, 109-120). Briefly, the assay was performed in the presence of (final concentration) 100mM triethanolamine buffer pH 8.5; 10mM magnesium acetate; 1mM EDTA; 30mM KCI; 1mM phosphoenolpyruvate (PEP); 2mM adenosinetriphosphate (ATP); 0.18mM nicotinamide-adenine-dinucleotide/reduced form (NADH); creatine kinase (CK); pyruvate kinase (PK), and lactate dehydrogenase (LDH). The concentration of creatine was determined from the oxidation of NADH measured spectrophotometrically at 340nm. $\text{CK: Cr + ATP → PCr + ADP}$$\text{PK: ADP + PEP → ATP + Pyruvate}$$\text{LDH: Pyruvate + NADH → Lactate + NAD}$
6. The results of the analyses are shown in Tables 1-4 below.

**Table 1**

| | **pH** | **Days of storage** | **g creatine monohydrate** per kg suspension |
|---|---|---|---|
| **Liquid honey** | 3.86 | 0 | 91.0 |
| | | 14 | 89.0 |
| | | 31 | 88.9 |
| | | 45 | 88.6 |
| | | 73 | 91.5 |

**Table 2**

| | pH | Days of storage | g creatine monohydrate **per kg suspension** |
|---|---|---|---|
| **Solid honey** | 3.87 | 0 | 90.7 |
| | | 14 | 90.3 |
| | | 31 | 90.7 |
| | | 45 | 89.9 |
| | | 73 | 88.6 |

**Table 3**

| | **pH** | **Days of storage** | **g creatine monohydrate per kg suspension** |
|---|---|---|---|
| **Maxim gel** | 2.05 | 0 | 91.5 |
| | | 14 | 90.1 |
| | | 31 | 87.4 |
| | | 45 | 88.0 |
| | | 73 | 86.8 |

**Table 4**

| | **pH** | **Days of storage** | **g creatine monohydrate per kg suspension** |
|---|---|---|---|
| **Syrup** | 5.13 | 0 | 84.6 |
| | | 14 | 86.3 |
| | | 31 | 86.0 |
| | | 45 | 86.1 |
| | | 73 | 84.3 |

### Results

There was no evidence of breakdown of creatine to creatinine during 73 days' (10.4 weeks') storage at normal room temperature in liquid and solid honey (pH 3.86) and syrup (pH 5.13). There was however a possible small loss of creatine of approximately 5% over the 73 days when suspended in Maxim Energy Gel, pH 2.05.

Creatine monohydrate suspended in Maxim Energy Gel, syrup and liquid honey formed a scum on the surface (i.e. floated to the top) which had to be well mixed in on sampling. This was least with the syrup. No scum was formed with the solid honey which formed a suspension most readily.

### CONCLUSIONS

When suspended, creatine is stable at room temperature at pHs where it would normally be degraded to creatinine if in solution.

To avoid the unsightly scum being formed it is suggested that creatine is suspended in spreads (such as cheese spread, caviar spread [popular in Scandinavia], smoked fish spread [also popular in Scandinavia - e.g. buckling paste]) contained in tubes. Since usually these are stored at temperatures below ambient (e.g. refrigerated at 4-6°C) this would further enhance stability upon storage.

### Example 2

This example describes the detailed formulation of an acidic composition in accordance with the invention.

The composition takes the form of a dry powder, which is to be suspended in an edible matrix to constitute a foodstuff comprising creatine and Aloe Vera extract.

| Ingredients | |
|---|---|
| Dextrose Monohydrate | 300g |
| Citric Acid | 32g |
| Pectin (stabilizer) | 6.0g |
| Salt | 5.0g |
| Trisodium Citrate | 5.0g |
| Beta Carotene | 3.0g |
| Potassium Chloride | 2.9g |
| Grapefruit Flavour | 2.9g |
| Tricalcium Phosphate | 2.1g |
| Heavy Magnesium Carbonate | 2.1g |
| Vitamin Premix | 1.8g |
| Lemon Flavour | 1.4g |
| Orange Flavour | 1.4g |
| Aspartame | 1.0g |
| Creatine Monohydrate | 88g |
| Lyophilized Aloe Vera extract | 7.6g |

About 63g of the above mixture when suspended in 1 litre of matrix provides, per 150ml serving, about 3g creatine, 0.6g Aloe Vera extract, (equivalent to 120ml juice), energy kJ 203 (kcal 48, assuming a zero calorie content for the supporting matrix), 11.1g carbohydrate, 156 mg chloride, 100mg sodium, 52mg potassium, 26mg calcium, 19.5mg magnesium, 13mg phosphorus, vitamins (vitamin E 3.4mg, vitamin C 16.2mg, Thiamin 0.3mg, Riboflavin 0.4mg, Niacin 5.0mg, vitamin B6 0.4mg, Folacin 85 g, vitamin B12 0.9 g, Biotin 0.08mg and Pantothenic acid 2.2mg) and traces of protein, fat, and fiber and has a pH of about 3.8.

### Example 3

This example relates to another embodiment of the invention.
The formulation is as in example 2 above, except that the 300g dextrose monohydrate is omitted and the aspartame content is increased to 2.5g to compensate. When 5.3g of this formulation is suspended in 250ml of matrix, it provides an almost calorie free food (assuming a non-calorific supporting matrix is used) containing creatine and electrolytes which is nutritionally useful to those wishing to lose or maintain their weight.

### Example 4

The pH of various foodstuffs was analysed, according to the method described in Example 1 above. The results are shown below. Each of these foodstuffs could potentially be used as an edible supporting matrix in a composition in accordance with the invention, by suspending therein a suitable amount of solid creatine. Suitable serving sizes (giving a creatine dose of between 1 and 5gms creatine) are also indicated.

| **Analysis of foodstuffs** | **pH** | **Serving (gms)** |
|---|---|---|
| Dolmio Sauce for Bolognese (original) | 3.97 | 200 |
| Uncle Ben's Rice Meal Maker (manufacturer M&M/Mars) | 4.33 | 200 |
| Sainsbury's economy Coleslaw | 4.22 | 200 |
| Heinz Ravioli in Tomato Sauce | 4.90 | 300 |
| Primula Savoury Spread | 5.80 | 30 |
| Primula Cheese Spread (with chives) | 6.07 | 30 |
| Sainsbury's Tuna and Mayonnaise Spread | 5.80 | 15 |
| Sainsbury's Beef Spread | 6.05 | 15 |

### Example 5

The object of this trial was to examine the stability of micronized creatine suspended in a matrix of Aloe Vera gel at room temperature for several weeks.

Aloe Vera gel containing 0.5% w/w Aloe Vera solid extract, and at pH 3.9, was obtained from Aloe Vera Industries Inc, Farmers Branch, Texas 75234 and micronized creatine from MW International Inc, Hillside, NJ 07205. To 100g gel was added differing quantities of micronized creatine, namely 2g, 5g and 10g. After the addition of the creatine the mixture was well stirred using a vortex mixer. An aliquot (5 ml) of each mixture was taken and kept frozen at -20°C. The remainder of the mixtures were stored at room temperature (approx 23°C) for several weeks and similar 5ml aliquots taken at 1, 2, 3, and 6 and 12 weeks and likewise frozen at -20°C. Before taking the aliquot, each mixture was well stirred using a vortex mixer.

At the end of the trial the aliquots were analysed for creatine as described in Example 1.

The results are shown in Table 5. Loss of creatine was highest in the 2g per 100g mixture and after 6 weeks approximately 36 % was lost. For the other two mixtures, (5g per 100g and 10g per 100g) the percentage losses were much less and at 6 weeks amounted to only 12% and 9.4% respectively. At 12 weeks the losses were 45%, 22% and 14% for the 2, 5, 10g per 100g mixtures respectively. These results are also represented graphically in Figure 1. The legend for the Figure is as follows: 2g/100g = square symbols; 5g/100g = shaded circles; 10g/100g = unshaded circles.

Although the micronized creatine remained suspended initially, after several days solid particles of micronized creatine were visible on the bottom of the vessel. It was concluded that at high concentrations of creatine in Aloe Vera gel (e.g. 5g per 100g and 10g per 100g) micronized creatine mixtures were substantially stable. However the settling out of the micronized creatine indicated that this preparation would be unsuitable for storage and use over several weeks.

**Table 5**

| Concentration of creatine (g/100g) in Aloe Vera gel after storage at room temperature, and (percentage creatine remaining). | | | | | | |
|---|---|---|---|---|---|---|
| Concentration of Creatine g/100g | Weeks | | | | | |
| | 0 | 1 | 2 | 3 | 6 | 12 |
| 2 | 2.05 | 1.69 | 1.54 | 1.43 | 1.32 | 1.12 |
| | (100%) | (82.6%) | (75.1%) | (69.6%) | (64.3%) | (55%) |
| | | | | | | |
| 5 | 4.99 | 4.84 | 4.69 | 4.57 | 4.00 | 3.90 |
| | (100%) | (97.0%) | (94.0%) | (91.5%) | (88.1%) | (78%) |
| | | | | | | |
| 10 | 10.2 | 10.07 | 9.80 | 9.56 | 9.28 | 8.82 |
| | (100%) | (98.4%) | (95.7%) | (93.3%) | (90.6%) | (86%) |

### Example 6

The object of this trial was to examine the stability of micronized creatine at room temperature for several weeks, when suspended in a composition having an edible matrix comprising Aloe Vera gel and either xanthan gum or carrageen gum.

Concentrated Aloe Vera gel and micronized creatine were obtained as described in Example 5. A solution of xanthan gum or carageen in the Aloe Vera gel was made by dissolving 2g solid gum in 100ml Aloe Vera gel by heating and stirring. To 100ml solution kept at 40°C was slowly added a slurry of 4g micronized creatine in 20ml water, and the mixture well stirred for 3 min using a vortex mixer to give a gel in which the micronized creatine was uniformly suspended. The whole mixture was subsequently cooled rapidly to room temperature when the suspension formed a semi-solid gel with a pH value of 5.0. Each mixture was kept at room temperature (23°C) for several weeks and an aliquot (5g) was taken at baseline (immediately after mixing) at 2 weeks and then again after 9 weeks. Creatine was analysed by the method described in Example 1.

The results are shown in Table 6. After 2 weeks for the carrageen matrix, the loss was 7.1 % and for xanthan gum 6.8%. After 9 weeks the loss was 21 % for both gums.

It is concluded that a suspension of creatine in Aloe Vera extract and edible gums, especially xanthan gum or carrageen, gives stability in concentrations of approximately 3g per 100g, even at acidic pH and storage at ambient temperature.

The mixture of the micronized creatine in each of the gums gave a perfect suspension and there was no tendency for solid micronized creatine to separate out to the bottom even after 9 weeks.

It is concluded that edible gums such as xanthan and carrageen provide an excellent supporting matrix for the presentation of creatine in a more stable semi-liquid form at room temperature over a period of many weeks, the gums being far more viscous than Aloe Vera gel.

**Table 6**

| Concentration of creatine (g/100g), and (% creatine remaining), in Aloe Vera gel mixed with two difference gums at room temperature. | | | |
|---|---|---|---|
| Concentration of creatine g/100g | Week | | |
| | 0 | 2 | 9 |
| Xanthan gum | 3.40 | 3.13 | 2.67 |
| | | (92%) | (79%) |
| | | | |
| Carrageen | 3.40 | 3.11 | 2.69 |
| | | (91%) | (79%) |

## Claims

1. A composition for human consumption, comprising solid creatine suspended in an edible viscous liquid or semi-liquid supporting matrix.

2. A composition according to claim 1, in which the composition has a pH in the range 2.5 to 6.5.

3. A composition according to claim 1 or 2, in which the composition has a pH in the range 3.0 to 6.0.

4. A composition according to any one of the preceding claims, comprising one or more additional components selected from the group consisting of: electrolytes, vitamins, lipids, carbohydrates, amino acids, trace elements, colorings, flavours, artificial sweeteners, natural health improving substances, antioxidants, stabilizers, preservatives and buffers.

5. A composition according to any one of the preceding claims, wherein the edible supporting matrix comprises one or more of the following: honey, syrup, molasses, treacle and concentrated Aloe Vera gel.

6. A composition according to any one of the preceding claims, wherein the edible supporting matrix comprises a plant-derived or algal-derived polysaccharide, gum, thickening agents, and gelatinous substances.

7. A composition accordance to any one of the preceding claims, wherein the edible supporting matrix comprises one or more of the following: galactomannans, dextrans, guar gum, locust bean gum, xanthan gum, carrageen, carrageenan, traganth gum, acacia gum, tara gum, gellan, gelatine, alginate, agar or pectin.

8. A composition according to any one of the preceding claims, wherein creatine is present in the form of creatine monohydrate or creatine phosphate.

9. A composition according to any one of the preceding claims, wherein a normal serving thereof provides a physiologically effective dose of creatine.

10. A composition according to any one of the preceding claims wherein a normal serving thereof provides 1-10 gms creatine.

11. An acidic composition according to any one of the preceding claims, wherein the creatine is substantially stable (with respect to conversion to creatinine) at 20-25°C for at least 7 days.

12. An acidic composition according to claim 11, wherein the creatine is substantially stable (with respect to conversion to creatinine) at 20-25°C for at least 31 days.

13. An acidic composition accordance to claim 11, wherein the creatine is substantially stable (with respect to conversion to creatinine) at 20-25°C for at least 45 days.

14. An acidic composition according to claim 11, wherein the creatine is substantially stable (with respect to conversion to creatinine) at 20-25°C for at least 73 days.

15. A method of preparing a composition for human consumption containing creatine in solid form in which the creatine is substantially stable, the method comprising the steps of: providing creatine in solid form; and mixing the solid creatine with an edible viscous liquid or semi-liquid supporting matrix so as to distribute the solid creatine within the supporting matrix.

16. A method according to claim 15, performance of which results in a composition according to any one of claims 1-14.

17. A method of storing creatine in stable form, the method comprising the steps of:
providing creatine in solid form; mixing the solid creatine with an edible viscous liquid or semi-liquid supporting matrix so as to suspend the creatine in solid form therein; and storing the suspended creatine at ambient temperature or below.

## Patentansprüche

1. Zusammensetzung für den menschlichen Verzehr, die festes Kreatin umfasst, das in einer essbaren, viskosen, flüssigen oder halbflüssigen tragenden Matrix suspendiert ist.

2. Zusammensetzung nach Anspruch 1, bei der die Zusammensetzung einen pH-Wert im Bereich von 2,5 bis 6,5 hat.

3. Zusammensetzung nach Anspruch 1 oder 2, bei der die Zusammensetzung einen pH-Wert im Bereich von 3,0 bis 6,0 hat.

4. Zusammensetzung nach einem der vorigen Ansprüche, die eine oder mehrere zusätzliche Komponenten umfasst, die aus der Gruppe ausgewählt werden, die aus Folgendem besteht: Elektrolyten, Vitamine, Lipide, Kohlehydrate, Aminosäuren, Spurenelemente, Farbstoffe, Geschmacksstoffe, künstliche Süßungsmittel, natürliche gesundheitsverbessernde Substanzen, Antioxidanzien, Stabilisatoren, Konservierungsstoffe und Puffer.

5. Zusammensetzung nach einem der vorigen Ansprüche, bei der die essbare tragende Matrix eines oder mehrere des Folgenden umfasst: Honig, Sirup, Melasse, Zuckersirup und konzentriertes Aloe Vera-Gel.

6. Zusammensetzung nach einem der vorigen Ansprüche, bei der die essbare tragende Matrix ein aus Pflanzen oder Algen gewonnenes Polysaccharid, Gummi, Verdickungsmittel und gelatineartige Substanzen umfasst.

7. Zusammensetzung nach einem der vorigen Ansprüche, bei der die essbare tragende Matrix eines oder mehrere des Folgenden umfasst: Galactomannane, Dextrane, Guargummi, Johannisbrotgummi, Xanthan, Carrageen, Carrageenan, Traganth-Gummi, Gummiarabikum, Taragummi, Gellan, Gelatine, Alginat, Agar-Agar oder Pektin.

8. Zusammensetzung nach einem der vorigen Ansprüche, bei der Kreatin in Form von Kreatinmonohydrat oder Kreatinphosphat vorhanden ist.

9. Zusammensetzung nach einem der vorigen Ansprüche, von der eine normale Portion eine physiologisch wirksame Dosis Kreatin liefert.

10. Zusammensetzung nach einem der vorigen Ansprüche, von der eine normale Portion 1-10 g Kreatin liefert.

11. Acidische Zusammensetzung nach einem der vorigen Ansprüche, bei der das Kreatin bei 20-25 °C wenigstens 7 Tage lang im Wesentlichen stabil ist (bezüglich einer Umwandlung in Kreatinin).

12. Acidische Zusammensetzung nach Anspruch 11, bei der das Kreatin bei 20-25 °C wenigstens 31 Tage lang im Wesentlichen stabil ist (bezüglich einer Umwandlung in Kreatinin).

13. Acidische Zusammensetzung nach Anspruch 11, bei der das Kreatin bei 20-25 °C wenigstens 45 Tage lang im Wesentlichen stabil ist (bezüglich einer Umwandlung in Kreatinin).

14. Acidische Zusammensetzung nach Anspruch 11, bei der das Kreatin bei 20-25 °C wenigstens 73 Tage lang stabil ist (bezüglich einer Umwandlung in Kreatinin).

15. Verfahren zur Herstellung einer Zusammensetzung für den menschlichen Verzehr, die Kreatin in fester Form enthält und in der das Kreatin im Wesentlichen stabil ist, wobei das Verfahren die Schritte umfasst: Vorsehen von Kreatin in fester Form und Mischen des festen Kreatins mit einer essbaren, viskosen, flüssigen oder halbflüssigen tragenden Matrix, so dass das feste Kreatin in der tragenden Matrix verteilt wird.

16. Verfahren nach Anspruch 15, dessen Ausführung zum Erhalt einer Zusammensetzung nach einem der Ansprüche 1 bis 14 führt.

17. Verfahren zur Lagerung von Kreatin in stabiler Form, wobei das Verfahren die Schritte umfasst: Vorsehen von Kreatin in fester Form, Mischen des festen Kreatins mit einer essbaren, viskosen, flüssigen oder halbflüssigen tragenden Matrix, so dass das Kreatin darin in fester Form suspendiert wird, und Lagern des suspendierten Kreatins bei Umgebungstemperatur oder darunter.

## Revendications

1. Composition destinée à la consommation humaine, comprenant de la créatine solide en suspension dans une matrice support comestible visqueuse liquide ou semi-liquide.

2. Composition selon la revendication 1, dans laquelle la composition a un pH compris entre 2.5 et 6.5.

3. Composition selon la revendication 1 ou 2, dans laquelle la composition a un pH compris entre 3.0 et 6.0.

4. Composition selon l'une quelconque des revendications précédentes, comprenant un ou plusieurs composants additionnels choisis dans le groupe constitué des électrolytes, vitamines, lipides, carbohydrates, acides aminés, oligoéléments, colorants, arômes, édulcorants artificiels, substances naturelles améliorant la santé, antioxydants, stabilisants, conservateurs et tampons.

5. Composition selon l'une quelconque des revendications précédentes, dans laquelle la matrice support comestible comprend un ou plusieurs des éléments suivants : miel, sirop, mélasses et gel d'aloès concentré.

6. Composition selon l'une quelconque des revendications précédentes, dans laquelle la matrice support comestible comprend un polysaccharide dérivé de plante ou d'algue, une gomme, des agents épaississants, et des substances gélatineuses.

7. Composition selon l'une quelconque des revendications précédentes, dans laquelle la matrice support comestible comprend un ou plusieurs des éléments suivants : des galactomannanes, dextranes, gomme de guar, farine de caroube, gomme de xanthane, mousse d'Irlande, carragénine, gomme de traganth, gomme arabique, gomme de tara, gellane, gélatine, alginate, agar ou pectine.

8. Composition selon l'une quelconque des revendications précédentes, dans laquelle la créatine est présente sous la forme de créatine monohydrate ou de créatine phosphate.

9. Composition selon l'une quelconque des revendications précédentes, dans laquelle une portion normale de celle-ci fournit une dose de créatine efficace sur le plan physiologique.

10. Composition selon l'une quelconque des revendications précédentes, dans laquelle une portion normale de celle-ci fournit 1-10 gms de créatine.

11. Composition acide selon l'une quelconque des revendications précédentes, dans laquelle la créatine est substantiellement stable (au regard de sa conversion en créatinine) à 20-25°C pendant au moins 7 jours.

12. Composition acide selon la revendication 11, dans laquelle la créatine est substantiellement stable (au regard de sa conversion en créatinine) à 20-25°C pendant au moins 31 jours.

13. Composition acide selon la revendication 11, dans laquelle la créatine est substantiellement stable (au regard de sa conversion en créatinine) à 20-25°C pendant au moins 45 jours.

14. Composition acide selon la revendication 11, dans laquelle la créatine est substantiellement stable (au regard de sa conversion en créatinine) à 20-25°C pendant au moins 73 jours.

15. Méthode de préparation d'une composition destinée à la consommation humaine contenant de la créatine sous forme solide dans laquelle la créatine est substantiellement stable, la méthode comprenant les étapes de : fournir la créatine sous forme solide ; et mélanger la créatine solide avec une matrice support comestible visqueuse liquide ou semi-liquide de manière à distribuer la créatine solide à l'intérieur de la matrice de support.

16. Méthode selon la revendication 15, dont l'exécution aboutit à une composition selon l'une quelconque des revendications 1-14.

17. Méthode de stockage de la créatine sous forme stable, la méthode comprenant les étapes de : fournir la créatine sous forme solide ; mélanger la créatine solide avec une matrice support comestible visqueuse liquide ou semi-liquide de manière à mettre la créatine sous forme solide en suspension dans celle-ci ; et stocker la créatine en suspension à température ambiante ou inférieure.
